# EUROPEAN PATENT SPECIFICATION

(11) **EP 2 405 263 B1**
(45) Date of publication and mention of the grant of the patent: **11.01.2017**
(21) Application number: 11171345.9
(22) Date of filing: 24.06.2011
(51) Int. Cl.: G01N 27/22, G01N 33/487

(54) **Analysis of a dielectric medium**
Analyse eines dielektrischen Mediums
Analyse d'un médium diélectrique

(30) Priority: 08.07.2010 GB 201011474
(43) Date of publication of application: 11.01.2012
(73) Proprietor: Aber Instruments Ltd, Ceredigion SY23 3AH (GB)
(72) Inventor: Todd, Robert, Powys, SY20 8QG (GB)
(74) Representative: Davies, Gregory Mark

(56) References cited:
- WO-A1-88/02115
- US-A- 3 665 302
- MORON Z ET AL: "Differential, three-electrode measurement of electrolytic conductivity", JOURNAL OF PHYSICS E. SCIENTIFIC INSTRUMENTS, IOP PUBLISHING, BRISTOL, GB, vol. 14, no. 6, 1 June 1981 (1981-06-01), pages 686-688, XP020016533, ISSN: 0022-3735, DOI: 10.1088/0022-3735/14/6/005

## Description

The present invention relates a method and apparatus for analysis of a dielectric medium.

In certain realisations the method and apparatus is for capacitance measurement in a dielectric medium, and in particular to such a system in which correction for polarisation error at measurement electrodes can be made.

Capacitance measurement techniques are known for measuring the capacitance (or specific capacitance or dielectric constant) of liquids and suspensions, such as biological cells in ionic aqueous solutions. Known techniques involve introducing metal electrodes into the liquid and applying an excitation signal (usually sinusoidal) and measuring voltage and current using a pair of measurement electrodes. The impedance, conductivity and specific capacitance can then be calculated. At high excitation frequencies (greater than about 1MHz) this is relatively straightforward with simple electrode and circuit configurations. However at lower frequencies, and particularly where the conductivity is high (up to 100mS/cm or so), the electrode -liquid interface exhibits an impedance which appears in series with the impedance of interest and distorts the measurements.

EP1018025 discloses such a technique for measurement of biomass. This document describes the background to the Beta dispersion and how correction needs to be made for polarisation at measurement electrodes.

Electrode polarisation effects result largely from the charged electrodes attracting around themselves a counter layer of ions which acts electrically as a capacitor/resistor network in series with the biomass suspension that is under measurement investigation. The magnitude of the electrode polarisation effect is largely frequency dependent. Unfortunately to be on the plateau of the Beta dispersion for meaningful biomass measurement requires the use of a frequency region in which polarisation of the measuring electrodes can contribute a significant capacitance which has a material distortion on the measured capacitance. This error also varies with time as the electrode surface impedance is not stable and depends upon electrode surface current density.

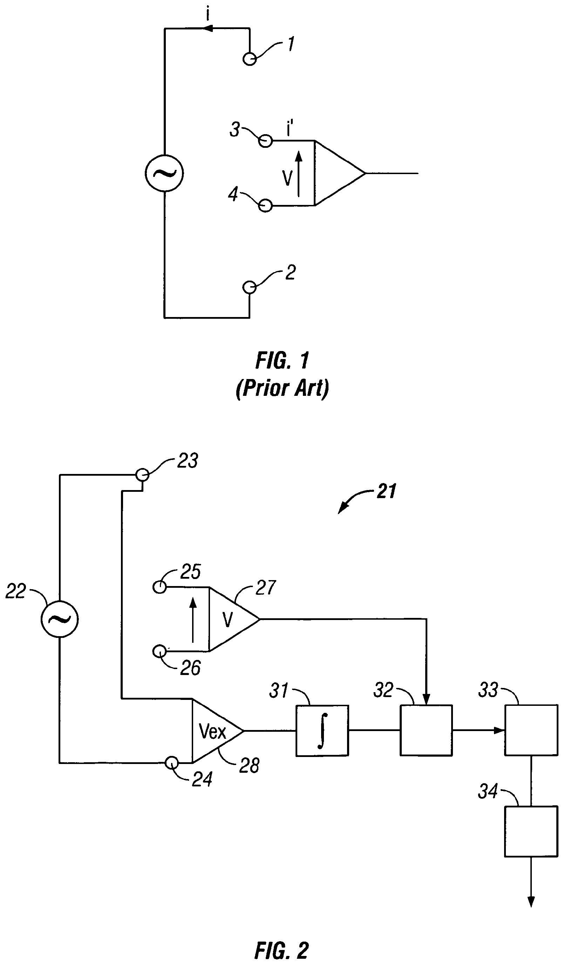

In a known prior art arrangement (shown in figure 1)a four pin electrode system is used in which two outer electrode pins 1,2 are used to drive current through the sample, whilst the two inner pins 3,4 are used to detect the potential drop (v) across the suspension between the inner pins 3,4. The potential is detected with a high impedance voltmeter, such that there is virtually no current i' flowing across the inner pins 3,4 electrode/solution interfaces. This minimises the polarisation effect at the measurement electrodes and hence the distortion in the capacitance measurement of the biomass medium. However such an arrangement does not remove the polarisation effect entirely, particularly in highly conductive media. Also, because of non-uniformity of the electrode fields and of electrode current density, the resulting measured voltage is not necessarily accurately representative of the voltage along all possible current paths.

A further technique for dealing with the electrode polarisation capacitance error is to estimate the error from the shape of the capacitance Vs frequency curve and then subtract the error from the measured values. This technique is described in EP1018025. This technique is possible because, at the lower end of the frequency range used for biological samples, the cell-suspension capacitance is usually relatively flat against frequency (plateau region) whereas the Electrode capacitance increases steeply as the frequency reduces. Whilst this technique works effectively in many circumstances, there are situations in which it is difficult to make an accurate estimate of the polarisation capacitance which is totally independent of the biomass characteristics.

There are other techniques for attempting to deal with the electrode polarisation error. Some of these are suitable for laboratory implementation but for various reasons not practicable for industrial on-line measurement. Electrode coatings designed to reduce the surface impedance can be effective but often do not have sufficiently long life effectiveness for typical operating conditions. Other methods such as variable geometry electrodes and differential methods using a reference electrode also have drawbacks, particularly for industrial on-line applications.

US3665302 A describes a method and apparatus for testing the condition of foods and due to the dielectric properties of some foods varying with time. The electrical quality factor and phase angle of a food sample are indicative of the amount of spoilage of the food. A probe comprising two pairs of electrodes is described for use in measuring the phase angle of a sample. The construction of the probe and the apparatus coupled thereto prevents interfacial polarisation where one pair of electrodes contact the sample, and hence one important source of error is overcome.

WO88/02115 A1 describes an apparatus for determination of biomass in a suspension. The apparatus comprises electrodes placed in a suspension where an alternating current voltage is applied between the electrodes. A current signal indicative of the current in a current electrode circuit is provided as is a means for providing a voltage signal indicative of the voltage between the voltage electrodes. Means for determining the ratio between the value of the voltage signal and the value of a quadrature component of the current signal or vice versa is provided to determine a capacitance dependent signal.

MORON Z, Differential, three-electrode measurement of electrolytic conductivity, Journal of Physics E, Scientific Instruments, IOP Publishing, Bristol, GB, Vol. 14, No.6, 1 June 1981, page 686-688, describes a manner of a differential method of electrolytic conductivity measurement and an electronic circuit for its use. This method enables the electrode polarisation influence on the measuring accuracy to be considerably reduced.

An improved technique and apparatus has now been devised.

According to a first aspect, the present invention provides a method for analysing dielectric test medium, as defined in claim 1.

In one embodiment, the second couple of electrodes may comprise one of the excitation electrodes and a sensing electrode. In such an embodiment, it is preferred that one of the excitation electrodes has a surface area significantly larger than the other of the excitation electrode couple. Beneficially, the larger surface excitation electrode comprises the excitation electrode in the second couple of electrodes. By significantly larger it is means that the surface area of the larger surface excitation electrode is at least 2 x (more preferably at least 5x, most preferably at least 10x) the surface area of the smaller surface area electrode.

In an alternative embodiment, separate respective couples of excitation electrodes and sensing electrodes are provided, the voltage being measured across the excitation electrode couple and the sensing electrode couple and compared in order to derive a polarisation correction factor. In this embodiment, the sensing electrodes comprise first and second sensing electrodes, neither being an excitation electrode.

A first couple of electrodes comprise first and second sensing electrodes (for example a pair of electrodes); and a second couple of electrodes comprise first and second excitation electrodes for applying the excitation current to the test medium, and the measured voltages across the excitation electrodes and the sensing electrodes are compared in order to derive a polarisation correction factor.

According to the invention, the voltage is therefore measured across the excitation electrodes in addition to being measured across the second electrode couple (for example the separate pair of sensing electrodes).

In one realisation of the invention, one couple of electrodes may comprise first and second sensing electrodes; and an alternate couple of electrodes may comprise first and second excitation electrodes for applying the excitation current to the test medium, and the measured voltages across the excitation electrodes and the sensing electrodes are compared in order to derive a polarisation correction factor.

Beneficially the measured voltages are used to derive a measure of the capacitance of the medium (typically using a capacitance value corrected for by the polarisation correction factor).

The voltages are complex values having phase components. The measured voltages between the first and second electrode couples are compared to reveal the out-of-phase component between them, which is then used to derive the polarisation correction factor.

Beneficially the quadrature component of the measured voltages of the electrode couples are compared to derive the polarisation correction factor.

It is preferred that the comparison comprises derivation of a ratio of measured values.

Beneficially the correction factor is applied to correct a value calculated from the measured voltage across one of the couples of electrodes.

In a preferred realisation, one couple of electrodes comprise first and second sensing electrodes; and another couple of electrodes comprise first and second excitation electrodes for applying the test current to the test medium; the correction factor being applied to correct a value calculated from the measured voltage across the sensing electrodes.

It is preferred that the voltage across one or both of the electrode couples is measured using a high impedance device such that there is negligible current flow (preferably less than 1 µA) across one or both of the electrode couples. This minimises the effect of the polarisation at the electrode surface.

Beneficially, the correction factor derived is used to provide a corrected value for capacitance, or conductivity.

In a preferred realisation, the test medium may be a biomass medium.

According to a further aspect, the present invention provides an apparatus for analysis of a dielectric test medium according to claim 8.

As described in relation to the first aspect, in one embodiment, one couple of electrodes may comprise first and second sensing electrodes; and the other couple of electrodes may comprise first and second excitation electrodes for applying the excitation current to the test medium, and the measured voltages across the excitation electrodes and the sensing electrodes may be compared in order to derive a polarisation correction factor.

In an alternative embodiment the first couple of electrodes may comprise an excitation electrode couple (preferably comprising first and second respective excitation electrodes). In this embodiment the second electrode couple comprises one of the excitation electrodes and a sensing electrode. Beneficially, one of the excitation electrodes has a surface area significantly larger than the other of the excitation electrode couple. The larger surface excitation electrode comprises the excitation electrode in the second couple of electrodes.

The invention will now be further described, by way of example only and with reference to the accompanying drawings, in which:
Figure 1 is a schematic representation of a prior art apparatus for use in capacitance measurement techniques;
Figure 2 is a schematic representation of apparatus for use in capacitance measurement correction techniques in accordance with the invention;
Figure 3 is a schematic view of an alternative embodiment of apparatus for use in capacitance measurement correction techniques in accordance with the invention;
Figure 4 is a representation of measured and corrected capacitance against frequency plots for various biological suspensions.

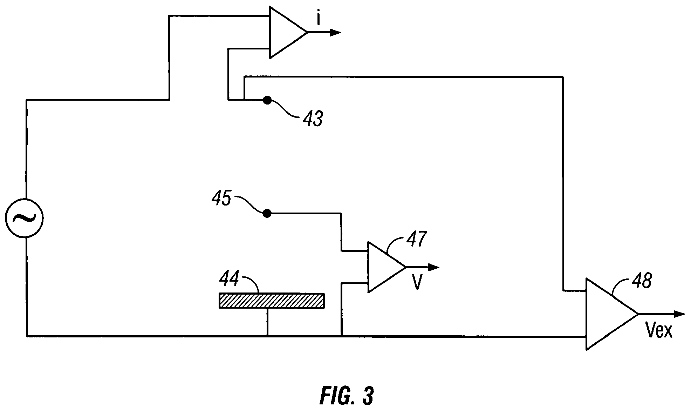

Referring to the drawings, and particularly figures 2 and 3, in a first embodiment, the present invention relies upon a four terminal electrode apparatus configuration 21 including a 1 voltage generator 22 which may be configured to generate a sinusoidal excitation voltage (although other excitation waveforms could be employed).

The two outer electrodes 23,24 are used to drive current through the sample, whilst the two inner electrodes 25,26 are used to detect the potential drop (v) across the suspension between the inner electrodes 25,26 . The potential is detected with a high impedance differential amplifier 27, such that there is virtually no current flowing across the inner electrodes 25, 26 electrode/solution interfaces. So far this is the same as with respect to the prior art configuration of figure 1. Importantly, in accordance with the invention, the excitation voltage Vex across the excitation electrodes 23, 24 is also measured by means of the differential amplifier 28.

At high excitation frequencies (greater than one to a few MHz) the voltage between the excitation electrode pair 23, 24 and the voltage between the voltage sensing pair 25, 26, can be related by a simple scalar constant which would depend upon the electrode geometry. This is because at the high excitation frequencies, the impedance of the suspension/electrode interface is negligibly small compared to that of the liquid or suspension. This scalar relationship is independent of the conductivity and permittivity of the liquid or suspension being measured. This is because it is determined by the ratio of impedances with identical phase versus frequency properties.

However at lower measuring frequencies, where the impedance of the suspension/electrode interface is not negligibly small compared to that of the liquid or suspension, voltage relationship departs from the high frequency value and becomes a complex quantity. Using the electrode arrangement of the invention as shown in figure 2 and in which the sensed voltage (v) is measured and compared with the measured voltage across the excitation electrodes 23, 24 the complex voltage ratio can be measured. The Voltage v and Vex will be in phase except for the effects of polarisation. The degree to which v and Vex are out of phase will be representative of the electrode polarisation error. By monitoring in particular the quadrature component of the out of phase complex voltage ratio of v and Vex, an estimate of the measurement error due to the electrode polarisation can be made. This can then be used to correct the main measured values of conductivity and specific capacitance.

The correction is derived at the same frequency as the specific capacitance or conductivity is measured, not extrapolated from a lower frequency measurement as is the case in respect of prior art techniques. This provides an improvement over prior art techniques.

The measured value of the voltages can readily be converted to values of conductivity or capacitance by means of known algorithms running on conventional processors or computer software.

Therefore, the output of amplifier 28 is fed via an integrator circuit 31 (to introduce a 90 degree phase shift) to a phase sensitive detector 32. The reference for the phase sensitive detector is taken from the output from the differential amplifier 27 measuring the excitation voltage. The output from the phase sensitive detector is passed via a low-pass filter 33 to a processor unit 34 which processes the signal to derive a value for the capacitance error due to the electrode polarisation layer. The function applied by the processor can be a linear function related to gain and offset, although more complex functions may be derived and utilised. The capacitance error is then subtracted from the measured capacitance to give the corrected capacitance.

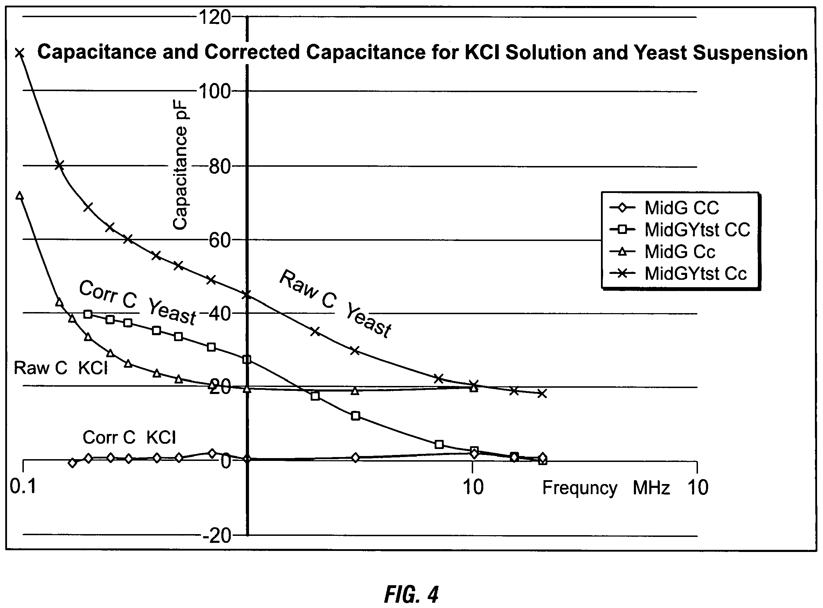

Figure 4 shows frequency and capacitance plots in respect of Yeast and KCI suspensions. The plots shown are in respect of raw measured capacitance derived from the measurement electrodes and also corrected data in which the correction factor was derived by means of comparison of the out of phase quadrature component of the voltage (V) across the measurement electrodes and the Voltage Vex across the excitation electrodes.

In an alternative technique in accordance with the invention a three electrode system may be used.

As shown in figure 3, the two outer electrodes 43,44 form a first electrode couple and are used to drive current through the sample. The surface area of electrode 44 is much larger than the surface area of the excitation electrode 43 and may be greater than five times larger or even greater than ten times larger. The electrode 44 could for example form a part of the container for the medium under investigation. The excitation voltage Vex across the excitation electrodes 43, 44 is measured by means of the differential amplifier 48.

In this embodiment a second electrode couple is formed by the large surface area excitation electrode 44 and the sensing electrode 45. This electrode couple is used to detect the potential drop (v) across the suspension between the inner electrodes 44 and 45. The potential is detected with a high impedance differential amplifier 47, such that there is virtually no current flowing.

In this embodiment, because of the large surface area of the excitation electrode 44, (with respect to the excitation electrode 43) the impedance of the electrode 44 (including the contribution due to surface polarisation) is negligible compared to that of the other excitation electrode, and that of the test medium. As a result the voltage ill be extremely close to that of the test medium adjacent the large electrode 44. The consequence of this is that, for practical purposes, the large electrode 44 can be used both as an excitation electrode and a sensing electrode. The signals are processed in the same manner as with respect to the earlier described embodiment.

## Claims

1. A method for analysing a dielectric test medium, the method comprising:
applying an excitation current to the test medium at a test frequency, by means of a first electrode couple (23, 24) comprising excitation electrodes;
measuring a voltage across the excitation electrode couple (23, 24);
measuring a voltage across a second couple of electrodes (25, 26);
**characterised in that** the method further comprises the step of:
comparing the measured voltages across the first and second electrode couples to determine an out of phase component between the measured voltages and deriving a polarisation correction factor using the out of phase component.

2. A method according to claim 1, wherein:
(i) the second couple of electrodes comprises one of the excitation electrodes (44) and a sensing electrode (43); and/or
(ii) one of the excitation electrodes (44) has a surface area greater than two times
larger than the other of the excitation electrode couple (43), preferably wherein the larger surface excitation electrode (44) comprises the excitation electrode in the second couple of electrodes.

3. A method according to claim 1, wherein:
(i) separate respective couples of excitation electrodes (23, 24) and sensing electrodes (25, 26) are provided, the voltage being measured across the excitation electrode couple and the sensing electrode couple are compared and a polarisation correction factor is derived; and/or
(ii) a second couple of electrodes (25, 26) comprise first and second sensing electrodes; and
a first couple of electrodes (23, 24) comprise first and second excitation electrodes for applying the excitation current to the test medium, and the measured voltages across the excitation electrodes and the sensing electrodes are compared in order to derive a polarisation correction factor.

4. A method according to any preceding claim, wherein:
the quadrature component of the measured voltages of the excitation (23, 24) and
second electrode couples (25, 26) are compared to derive the polarisation correction factor.

5. A method according to any preceding claim wherein the correction factor is applied to correct a value calculated from the measured voltage across one of the couples of electrodes.

6. A method according to claim 5, wherein the second couple of electrodes comprise
first and second sensing electrodes (25, 26) ; the correction factor being applied to correct a value calculated from the measured voltage across the sensing electrodes.

7. A method according to any preceding claim, wherein:
(i) the voltage across one or both of the electrode couples is measured using a
high impedance device (27) such that there is negligible current flow, preferably less than 1 µA, across the second electrode couple; and/or
(ii) the correction factor derived is used to provide a corrected value for capacitance, or conductivity; and/or
(iii) the test medium is a biomass medium.

8. Apparatus (21) for analysis of a dielectric test medium, the apparatus comprising:
a first couple of electrodes (23, 24) capable of being disposed in the test medium;
a second couple of electrodes (25, 26) capable of being disposed in the test medium;
means (22) for applying an excitation current to the test medium at a test frequency, by means of the first couple of electrodes;
means (28) for measuring a voltage across the first couple of electrodes;
means (27) for measuring a voltage across the second couple of electrodes;
**characterised in that** the apparatus further comprises: processing means (34) configured for comparing the measured voltages across the first (23, 24) and second (25, 26) electrode couples to determine an out of phase component between the measured voltages and deriving a polarisation correction factor using the out of phase component.

9. Apparatus according to claim 8, wherein the first couple of electrodes (23, 24) comprise an excitation electrode couple.

10. Apparatus according to claim 9, wherein the excitation electrode couple (23, 24) comprises first and second respective excitation electrodes.

11. Apparatus according to any of claims 8 to 10, wherein the second electrode couple comprises one of the excitation electrode; (44) and a sensing electrode (43).

12. Apparatus according to any of claims 8 to 11, wherein one of the excitation electrodes (44) has a surface area greater than two times larger than the other of the excitation electrode couple (43).

13. Apparatus according to claim 12, wherein the larger surface excitation electrode (44) comprises the excitation electrode in the second couple of electrodes.

14. Apparatus according to any of claims 8 to 10, wherein separate respective couples of excitation electrode; (23, 24) and sensing electrode; (25, 26) are provided.

15. Apparatus according to any of claims 8 to 14, wherein the voltage across one or both of the electrode couples is measured using a high impedance device (27) such that there is negligible current flow, preferably less than 1 µA across one or both of the electrode couples.

## Patentansprüche

1. Verfahren zum Analysieren eines dielektrischen Testmediums, wobei das Verfahren Folgendes umfasst:
Anlegen eines Erregungsstroms mit einer Testfrequenz an das Testmedium mittels eines ersten Elektrodenpaares (23, 24), das Erregungselektroden umfasst;
Messen einer Spannung über dem Erregungselektrodenpaar (23, 24);
Messen einer Spannung über einem zweiten Elektrodenpaar (25, 26);
**dadurch gekennzeichnet, dass** der Verfahren ferner den folgenden Schritt umfasst:
Vergleichen der gemessenen Spannungen über dem ersten und dem zweiten Elektrodenpaar, um eine phasenverschobene Komponente zwischen den gemessenen Spannungen zu bestimmen, und Ableiten eines Polarisationskorrekturfaktors unter Verwendung der phasenverschobenen Komponente.

2. Verfahren nach Anspruch 1, wobei:
(i) das zweite Elektrodenpaar eine der Erregungselektroden (44) und eine Messelektrode (43) umfasst; und/oder
(ii) eine der Erregungselektroden (44) eine Oberfläche aufweist, die mehr als zwei Mal so groß wie die andere Elektrode des Erregungselektrodenpaares (43) ist, wobei die Erregungselektrode (44) mit der größeren Oberfläche vorzugsweise die Erregungselektrode in dem zweiten Elektrodenpaar umfasst.

3. Verfahren nach Anspruch 1, wobei:
(i) jeweils getrennte Paare von Erregungselektroden (23, 24) und Messelektroden (25, 26) vorgesehen sind, wobei die Spannungen, die über dem Erregungselektrodenpaar und dem Messelektrodenpaar gemessen werden, verglichen werden und ein Polarisationskorrekturfaktor abgeleitet wird; und/oder
(ii) ein zweites Elektrodenpaar (25, 26) eine erste und eine zweite Messelektrode umfasst; und ein erstes Elektrodenpaar (23, 24) eine erste und eine zweite Erregungselektrode umfasst, um den Erregungsstrom an das dielektrische Testmedium anzulegen, und wobei die über den Erregungselektroden und den Messelektroden gemessenen Spannungen verglichen werden, um einen Polarisationskorrekturfaktor abzuleiten.

4. Verfahren nach einem der vorhergehenden Ansprüche, wobei:
die Quadraturkomponenten der gemessenen Spannungen der Erregungselektroden (23, 24) und des zweiten Elektrodenpaars (25, 26) verglichen werden, um den Polarisationskorrekturfaktor abzuleiten.

5. Verfahren nach einem der vorhergehenden Ansprüche, wobei der Korrekturfaktor angewendet wird, um einen Wert zu korrigieren, der aus der über einem der Elektrodenpaare gemessenen Spannung berechnet worden ist.

6. Verfahren nach Anspruch 5, wobei das zweite Elektrodenpaar eine erste und eine zweite Messelektrode (25, 26) umfasst; wobei der Korrekturfaktor angewendet wird, um einen Wert zu korrigieren, der aus der über den Messelektroden gemessenen Spannung berechnet worden ist.

7. Verfahren nach einem der vorhergehenden Ansprüche, wobei:
(i) die Spannung über einem oder beiden Elektrodenpaare unter Verwendung einer Einrichtung (27) mit hoher Impedanz gemessen wird, so dass ein vernachlässigbarer Stromfluss, vorzugsweise kleiner als 1 µA, auftritt; und/oder
(ii) der abgeleitete Korrekturwert verwendet wird, um einen korrigierten Wert für die Kapazität oder für die Leitfähigkeit bereitzustellen; und/oder
(iii) das Testmedium ein Biomassemedium ist.

8. Vorrichtung (21) für die Analyse eines dielektrischen Testmediums, wobei die Vorrichtung Folgendes umfasst:
ein erstes Elektrodenpaar (23, 24), das in dem Testmedium angeordnet werden kann;
ein zweites Elektrodenpaar (25, 26), das in dem Testmedium angeordnet werden kann;
Mittel (22) zum Anlegen eines Erregungsstroms mit einer Testfrequenz an das Testmedium mittels des ersten Elektrodenpaares;
Mittel (28) zum Messen einer Spannung über dem ersten Elektrodenpaar;
Mittel (27) zum Messen einer Spannung über dem zweiten Elektrodenpaar;
**dadurch gekennzeichnet, dass** die Vorrichtung ferner Folgendes umfasst:
Verarbeitungsmittel (34), die konfiguriert sind, die über dem ersten (23, 24) und dem zweiten (25, 26) Elektrodenpaar gemessenen Spannungen zu vergleichen, um eine phasenverschobene Komponente zwischen den gemessenen Spannungen zu bestimmen, und unter Verwendung der phasenverschobenen Komponente einen Polarisationskorrekturfaktor abzuleiten.

9. Vorrichtung nach Anspruch 8, wobei das erste Elektrodenpaar (23, 24) ein Erregungselektrodenpaar umfasst.

10. Vorrichtung nach Anspruch 9, wobei das Erregungselektrodenpaar (23, 24) jeweils eine erste und eine zweite Erregungselektrode umfasst.

11. Vorrichtung nach einem der Ansprüche 8 bis 10, wobei das zweite Elektrodenpaar eine der Erregungselektroden (44) und eine Messelektrode (43) umfasst.

12. Vorrichtung nach einem der Ansprüche 8 bis 11, wobei eine der Erregungselektroden (44) eine Oberfläche aufweist, die größer als zwei Mal so groß wie die andere Elektrode des Erregungselektrodenpaares (43) ist.

13. Vorrichtung nach Anspruch 12, wobei die Erregungselektrode (44) mit der größeren Oberfläche die Erregungselektrode in dem zweiten Elektrodenpaar umfasst.

14. Vorrichtung nach einem der Ansprüche 8 bis 10, wobei jeweils getrennte Paare von Erregungselektroden (23, 24) und Messelektroden (25, 26) vorgesehen sind.

15. Vorrichtung nach einem der Ansprüche 8 bis 14, wobei die Spannung über einem oder beiden Elektrodenpaaren unter Verwendung einer Einrichtung (27) mit hoher Impedanz gemessen wird, so dass über einem oder beiden Elektrodenpaaren ein vernachlässigbarer Stromfluss, vorzugsweise kleiner als 1 µA, auftritt.

## Revendications

1. Procédé d'analyse d'un milieu d'essai diélectrique, le procédé comprenant :
l'application d'un courant d'excitation au milieu d'essai à une fréquence d'essai, au moyen d'un premier couple d'électrodes (23, 24) comprenant des électrodes d'excitation ;
la mesure d'une tension aux bornes du couple d'électrodes d'excitation (23, 24) ;
la mesure d'une tension aux bornes d'un deuxième couple d'électrodes (25, 26) ;
**caractérisé en ce que** le procédé comprend en outre l'étape consistant à :
comparer les tensions mesurées aux bornes des premier et deuxième couples d'électrodes pour déterminer une composante de déphasage entre les tensions mesurées et déduire un facteur de correction de polarisation en utilisant la composante de déphasage.

2. Procédé selon la revendication 1, dans lequel :
(i) le deuxième couple d'électrodes comprend une des électrodes d'excitation (44) et une électrode de détection (43) ; et/ou
(ii) une des électrodes d'excitation (44) a une surface plus de deux fois plus grande que l'autre électrode du couple d'électrodes d'excitation (43),
de préférence dans lequel l'électrode d'excitation de plus grande surface (44) constitue l'électrode d'excitation dans le deuxième couple d'électrodes.

3. Procédé selon la revendication 1, dans lequel :
(i) des couples respectifs distincts d'électrodes d'excitation (23, 24) et d'électrodes de détection (25, 26) sont mis en oeuvre, les tensions mesurées aux bornes du couple d'électrodes d'excitation et du couple d'électrodes de détection sont comparées et un facteur de correction de polarisation est déduit ; et/ou
(ii) un deuxième couple d'électrodes (25, 26) comprend des première et deuxième électrodes de détection ; et
un premier couple d'électrodes (23, 24) comprend des première et deuxième électrodes d'excitation destinées à appliquer le courant d'excitation au milieu d'essai, et les tensions mesurées aux bornes des électrodes d'excitation et des électrodes de détection sont comparées afin de déduire un facteur de correction de polarisation.

4. Procédé selon une quelconque revendication précédente, dans lequel :
les composantes déphasées des tensions mesurées du couple d'électrodes d'excitation (23, 24) et du deuxième couple d'électrodes (25, 26) sont comparées pour déduire le facteur de correction de polarisation.

5. Procédé selon une quelconque revendication précédente dans lequel le facteur de correction est appliqué pour corriger une valeur calculée à partir de la tension mesurée aux bornes d'un des couples d'électrodes.

6. Procédé selon la revendication 5, dans lequel le deuxième couple d'électrodes comprend des première et deuxième électrodes de détection (25, 26) ;
le facteur de correction étant appliqué pour corriger une valeur calculée à partir de la tension mesurée aux bornes des électrodes de détection.

7. Procédé selon une quelconque revendication précédente, dans lequel :
(i) la tension aux bornes d'un ou des deux couples d'électrodes est mesurée en utilisant un dispositif de forte impédance (27) de telle sorte qu'un courant négligeable, de préférence inférieur à 1 µA, circule à travers le deuxième couple d'électrodes ; et/ou
(ii) le facteur de correction déduit est utilisé pour fournir une valeur corrigée de capacité ou de conductivité ; et/ou
(iii) le milieu d'essai est un milieu de biomasse.

8. Appareil (21) destiné à l'analyse d'un milieu d'essai diélectrique, l'appareil comprenant :
un premier couple d'électrodes (23, 24) susceptibles d'être disposées dans le milieu d'essai ;
un deuxième couple d'électrodes (25, 26) susceptibles d'être disposées dans le milieu d'essai ;
un moyen (22) pour appliquer un courant d'excitation au milieu d'essai à une fréquence d'essai, au moyen du premier couple d'électrodes ;
un moyen (28) pour mesurer une tension aux bornes du premier couple d'électrodes ;
un moyen (27) pour mesurer une tension aux bornes du deuxième couple d'électrodes ;
**caractérisé en ce que** l'appareil comprend en outre :
un moyen de traitement (34) configuré pour comparer les tensions mesurées aux bornes des premier (23, 24) et deuxième (25, 26) couples d'électrodes pour déterminer une composante de déphasage entre les tensions mesurées et déduire un facteur de correction de polarisation en utilisant la composante de déphasage.

9. Appareil selon la revendication 8, dans lequel le premier couple d'électrodes (23, 24) constitue un couple d'électrodes d'excitation.

10. Appareil selon la revendication 9, dans lequel le couple d'électrodes d'excitation (23, 24) comprend des première et deuxième électrodes d'excitation respectives.

11. Appareil selon l'une quelconque des revendications 8 à 10, dans lequel le deuxième couple d'électrodes comprend une des électrodes d'excitation (44) et une électrode de détection (43).

12. Appareil selon l'une quelconque des revendications 8 à 11, dans lequel une des électrodes d'excitation (44) a une surface plus de deux fois plus grande que l'autre électrode du couple d'électrodes d'excitation (43).

13. Appareil selon la revendication 12, dans lequel l'électrode d'excitation de plus grande surface (44) constitue l'électrode d'excitation dans le deuxième couple d'électrodes.

14. Appareil selon l'une quelconque des revendications 8 à 10, dans lequel des couples respectifs distincts d'électrodes d'excitation (23, 24) et d'électrodes de détection (25, 26) sont mis en oeuvre.

15. Appareil selon l'une quelconque des revendications 8 à 14, dans lequel la tension aux bornes d'un ou des deux couples d'électrodes est mesurée en utilisant un dispositif de forte impédance (27) de telle sorte qu'un courant négligeable, de préférence inférieur à 1 µA, circule à travers un ou les deux couples d'électrodes.
